# EUROPEAN PATENT APPLICATION

(11) **EP 4 707 402 A1**
(43) Date of publication of application: **11.03.2026**
(21) Application number: 24199436.7
(22) Date of filing: 10.09.2024
(51) Int. Cl.: C12P 7/02, C12N 9/02, C12P 33/06, C25B 11/085

(54) **BIOELECTROCHEMICAL HYDROXYLATION OF SUBSTRATES USING FUNCTIONAL WATER-DEPENDENT ALKYL-HYDROXYLASE HYBRID ELECTRODES AND RESPECTIVE BIOFILM HYBRID ELECTRODES**

(71) Applicant: Albert-Ludwigs-Universität Freiburg Körperschaft des öffentlichen Rechts, 79098 Freiburg (DE)
(72) Inventor: Fischer, Anna, 79117 Freiburg (DE); Boll, Matthias, 79194 Gundelfingen (DE); Ortlieb, Niklas, 79108 Freiburg (DE); Kosian, Dennis, 79111 Freiburg (DE); Weßbecher, Ralf, 79106 Freiburg (DE); Davis, Victoria, 8302 Kloten (CH)
(74) Representative: Krauss, Jan

(57) **Abstract**

The present invention relates to methods for the bioelectrochemical hydroxylation of substrates with functional water-dependent alkyl hydroxylase-hybrid electrodes and/or respective biofilm hybrid electrodes.

## Description

The present invention relates to methods for the bioelectrochemical hydroxylation of substrates with functional water-dependent alkyl hydroxylase-hybrid electrodes and/or respective biofilm hybrid electrodes.

### Background of the invention

In humans, vitamin D₃ (VitD₃, cholecalciferol) is an essential vitamin that can be formed photochemically in the skin from the precursor 7-dehydrocholesterol (DHC) (Lips 2006). Classically, a deficiency of VitD₃ is associated with osteomalacia and rickets. Today, a series of additional diseases are associated with a vitamin D-deficiency, such as respiratory diseases (including Covid-19), complications during pregnancy, and even cancer and diabetes.

In particular in the northern hemisphere, VitD₃ deficiency is a pandemic. Amongst other factors, this is caused by a reduced exposition to sunlight, impeding the endogenous synthesis of VitD₃ from 7-dehydrocholesterol (DHC) under UV-light (Pilz et al., 2018). In livestock breeding, VitD₃ deficiency is particularly pronounced under artificial light conditions, which negatively influences bone formation and causes substantial impediments of motility.

Vitamin D₃ itself is biologically inactive and is metabolized in the liver by cytochrome P450 (CYP) enzymes into the circulating storage form 25-OH-VD₃. Thus, the level of serum 25-OH-VitD₃ is an indicator for the VitD-status in the body. Following another hydroxylation in the kidney, the actual and highly effective hormone calcitriol is generated. Supplementation of food by 25-OH-VitD3 results in much higher 25-OH-VitD₃ serum concentrations than the VitD₃ precursor. For this reason, 25-OH-VitD₃ has to be viewed as a much more effective food supplement for the prevention and treatment of human diseases associated with VitD-deficiency (Cashman et al., 2012). Moreover, the addition of 25-OH-VitD₃ exhibits significant positive effects in farming of pigs and poultry, in particular a strengthening of bone formation and a reduction of diseases caused by VitDs-deficiency (Wideman et al., 2015, Thayer et al. 2019). The preparation Hy-D^{®} comprising 25-OH-VitD₃ as active ingredient is, for example, commercialized by the company DSM.

In analogy to the conversion in the skin, starting from 7-DHC, VitD₃ can be obtained through UV-irradiation in established methods (Escribà-Gelonch et al., 2018). Currently published methods for the conversion of 7-DHC or VitD₃ into the C25-hydroxylated form employ heterologously produced cytochrome P450 (CYP) enzymes (as examples: Kang et al. 2015, Abdulmughni et al. 2017, Tang et al. 2020) as bio-catalysts, or chemical synthesis methods (Zhu and Okumara, 1995). In principle, enzymatic methods are superior to chemical methods with respect to selectivity and yield, whereas chemical methods do not involve enzyme catalysts that are only stable within a small range of solvent-, pH, and temperature. Current biocatalytic methods for the synthesis of 25-OH-VitD₃ from VitD₃ by means of microbial CYP monooxygenases depend on the co-substrate O₂ and additional biological electron donor systems comprising NAD(P)H and a NAD(P)H dependent dehydrogenase. Such systems, in turn, require the use of additional enzymatic systems for the regeneration of the electron donor. Systems as described exhibit yield at a maximum of 74.4% (Tang et al. 2020). An essential problem is the lack of selectivity of CYP monooxygenases. In particular the production of 1,25-dihydroxy-VitD₃, the highly potent hormonal form of VitD₃, is problematic and requires cost-intensive methods for separation.

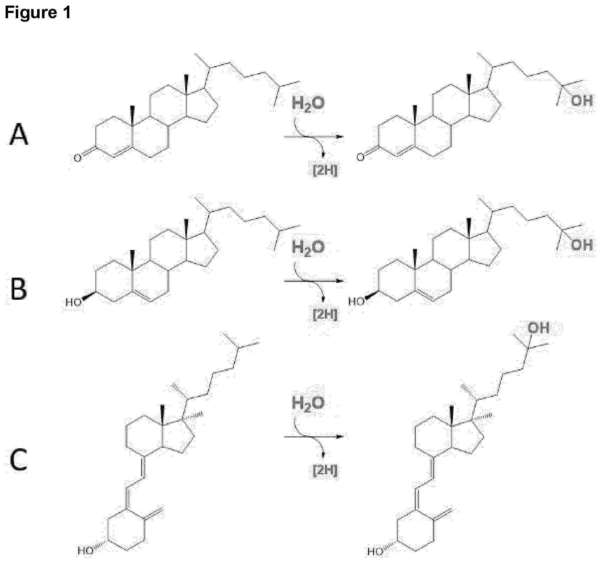

The beta-proteobacterium *Sterolibacterium denitrificans* is able to metabolize steroids as the sole carbon and energy source, such as cholesterol, under denitrifying conditions with nitrate instead of oxygen as terminal electron acceptor of an anaerobic respiratory chain. On the basis of polyphasic evidence, the bacterial strain Chol-1S(T) (=DSM 13999T=ATCC BAA-354T) was proposed as the type-strain for *Sterolibacterium denitrificans.* During growth with cholesterol, the bacterium produces oxygen-independent, molybdenum-cofactor- containing hydroxylases that use water as hydroxylating co-substrate and an electron acceptor, instead of common CYP monooxygenases. During the degradation of cholesterol, the enzyme steroid C25 dehydrogenase 1 (S25DH1) catalyzes the C25-hydroxylation of the intermediate cholest-4-ene-3-one (Figure 1A).

The prototypic enzyme S25DH1 was isolated and characterized from the wildtype strain of *S*. *denitrificans* (Dermer et al., 2012). The catalytic reaction takes place at a molybdenum-cofactor bound to the A-subunit of the enzyme. The B- and C- subunits provide an electron transfer chain to the natural acceptor cytochrome c. A chaperone, also imprecisely designated as D-subunit, is important for the folding of the trimeric enzyme complex, and the insertion of the molybdenum-cofactor. S25DH1 belongs to a class of related molybdoenzymes having the same structure and cofactor equipment, that all hydroxylate C-H-residues in tertiary, secondary or primary-allylic or primary-benzylic position using water as hydroxyl donor. S25DH1 hydroxylates the tertiary C25 of the side chain of cholest-4-ene-3-one with a high specificity (>99%). Nevertheless, for continuous reactions and/or operation, a steady supply of fresh electron acceptor must be maintained, or the acceptor has to be regenerated.

It was found earlier in 2016 that cellular extracts of *S*. *denitrificans,* when grown on cholesterol, in addition to the natural substrate cholest-4-ene-3-one, in a highly specific and complete (>99%) fashion convert both DHC as well as VitD₃ into the 25-OH-products (Figure 1), in the same manner as the enzyme S25DH1 when purified from cell extracts. The conversion of VitD₃ additionally requires at least 5% 2-hydroxypropyl-betacyclodextrin, in order to move the equilibrium into the direction of pre-VitD₃, the actual substrate of S25DH1 (Warnke et al., 2016).

*Thauera aromatica* K172 (DSM 6984) is a facultatively anaerobic, mesophilic beta-proteobacterium that was isolated from sewage sludge (Anders HJ, et al. 1995). When grown with organic compounds such as benzoate, it can use nitrate as electron acceptor in an anaerobic respiratory chain, thereby employing a molybdenum-cofactor containing nitrate reductase related to S25DHs.

Due to the above-mentioned limitations of enzymatic and chemical procedures there is still a need in the art for new approaches to convert of VitD₃, DHC and analogous alkyl-substrates into 25-OH-VitD₃, 25-OH-DHC or analogous hydroxylated products.

It is therefore an object of the present invention to provide new methods for the bioelectrochemical hydroxylation of organic compounds using water-dependent alkyl-hydroxylases, and in particular to produce the tertiary alcohol 25-OH-VitD₃, 25-OH-DHC or analogous hydroxylated products using S25DH enzyme and *Thauera aromatica* K172 whole-cells, which overexpresses the enzyme. Other objects and advantages of the present invention can be readily derived by the person of skill when studying the following description in detail.

In a first aspect thereof, the present invention solves the above object by providing a method for the bioelectrochemical hydroxylation of an organic compound, comprising a) providing at least one biocompatible electrode, b) immobilizing at least one suitable water-dependent alkyl-hydroxylase on the material of the biocompatible electrode, c) providing at least one suitable substrate to be hydroxylated by the water-dependent alkyl-hydroxylase, d) suitably contacting the at least one substrate of c) with the immobilized water-dependent alkyl-hydroxylase of b), and enzymatically hydroxylating the substrate comprising an electron transfer between enzyme and electrode, preferably a direct electron transfer, and e) optionally, isolating said hydroxylated substrate.

In a second aspect thereof, the present invention solves the above object by providing a method for the bioelectrochemical hydroxylation of an organic compound, comprising a) providing at least one biocompatible electrode, b1) providing a suspension of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell, wherein the suspension preferably comprises resting cells of a cell density (OD) of between about 10 and about 100, preferably about 50, or b2) suitably providing a biofilm, preferably a biofilm grown directly on the at least one biocompatible electrode, of the at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell on the at least one biocompatible electrode, c) providing at least one suitable substrate to be hydroxylated by the water-dependent alkyl-hydroxylase, d) suitably contacting the at least one substrate of c) with the bacterium of b1) and/or b2), and enzymatically hydroxylating the substrate comprising an electron transfer between enzyme and electrode, and e) optionally, isolating said hydroxylated substrate.

PL226816B1 discloses the use of the enzyme C25DH1 from *Sterolibacterium denitrificans* wildtype either in purified form or present in a cellular extract only for the conversion of DHC into 25-OH-DHC.

WO 2022/101282A1 relates to methods using recombinant *Thauera aromatica* cells to produce oxygen-independent molybdenum-hydroxylases in order to convert vitamin D₃, 7-dehydrocholesterol and related alkylated compounds into 25-hydroxy-vitamin D₃, 25-hydroxy-7-dehydrocholesterol and analogous alcohols. The underlying biocatalytic method links the water-dependent hydroxylation of alkyl residues with the nitrate-reducing respiratory chain of the strain as electron acceptor system. In contrast to WO 2022/101282A1, the present method does no longer require the artificial regeneration of the electron-carrier. Furthermore, in WO 2022/10128A1 the regio-selective conversion of VitD₃ to 250HVitD₃ is dependent on the addition of the solubilizer HPCD and NO₃⁻ as the electron acceptor. The application generally mentions to use bacterial biofilms on electrodes; however, no teaching was provided how to develop and to grow usable biofilms. In the context of the present invention, the present inventors were the first to devise a strategy to successfully form biofilms of *Thauera aromatica* K172 on the electrode, which required specific materials and growth conditions in order to become both possible and efficient.

Most of the previously published studies on the biocatalytic conversion of VitD₃ to 250HVitD₃ are based on oxygen-dependent CYP monooxygenases. Apart from the necessity for costly NAD(P)H-regenerating systems, these platforms often lead to the formation of undesired side-products, in particular the hormonally active calcitriol, by introducing a second hydroxyl group at the C1 position. The formation of such by-products requires time-consuming and cost-intensive purification procedures. Biocatalytic systems based on S25DH (isolated enzymes or in whole cells) without an electrode as the final electron acceptor or electrochemical regeneration of the electron acceptor molecules are severely limited by the stoichiometric consumption of the electron acceptor during the hydroxylation of VitD₃, as the electron acceptor must be continuously replenished to enable a continuous and complete conversion.

By coupling either the isolated enzyme or the S25DH-producing *Thauera aromatica* K172 whole-cell biocatalyst, either in suspension or as a biofilm, with biocompatible inorganic electrodes, unique biohybrid electrodes that can continuously synthesize 250HVitD₃ are accessible. As a result, turnover limitation due to electron acceptor consumption can be overcome by a direct or mediated electron transfer towards the electrode as the final electron acceptor.

Preferred is the method according to the present invention, wherein water is used as hydroxylating agent.

In one aspect thereof, the present invention solves the above object by providing a method according to the present invention, wherein said method is performed with the recombinant strain in the form of a biofilm, preferably a biofilm on an electrode material.

In the present invention, as a preferred embodiment, two types of biohybrid electrodes for the highly specific bioelectrocatalytic conversion of VitD₃ to 25OHVitD₃ are provided, that overcome the previously mentioned limitations.
(a) Coupling heterologously produced and enriched S25DH of *Sterolibacterium denitrificans* with a suitable biocompatible electrode resulted in an enzyme biohybrid electrode with mainly direct electron transfer from enzyme to electrode. Direct electron transfer enables electron mediator independent continuous VitD₃ hydroxylation. Nevertheless, the addition of small amounts of electron mediator is still possible to increase the conversion of VitD₃ to 25OHVitD₃. Advantageously, only low concentrations of electron mediator are required since the mediator can be regenerated at the electrode.
(b) By using whole *Thauera aromatica* K172 cells that heterologously produce S25DH as a whole-cell biocatalyst, preferably in the periplasm thereof, VitD₃ -hydroxylating hybrid electrodes were provided. These couple the S25DH-producing *Thauera aromatica* K172 cells, in suspension or as biofilms, with a suitable biocompatible electrode. Cell suspensions with high optical density can be used with low concentrations of electron mediator for the transfer of electrons from the periplasm-located S25DH to the electrode. In addition, S25DH-producing cells can be grown as a biofilm directly onto the electrode, reducing the distance for diffusive mediated electron transfer and further increasing the efficiency. As a result, 250HVitD₃ is continuously produced without limitation by the mediator concentration since the mediator is efficiently regenerated at the electrode.

Preferred is the method according to the present invention, wherein the water-dependent alkyl-hydroxylase is obtained from *Sterolibacterium denitrificans,* preferably *Sterolibacterium* Chol-1-S steroid C25 dehydrogenase (S25DH1-4) and steroid C26 dehydrogenase (S26DH), *Aromatoleum aromaticum* preferably *Aromatoleum aromaticum* EbN1 ethylbenzene dehyrogenase (EbDH) or *Aromatoleum aromaticum* pCyN1 para-cymene dehydrogenase (CmdABC) and *Desulfococcus oleovorans* preferably *Desulfococcus oleovorans* Hxd3 alkane hydroxylase (AhyABC), preferably from *Sterolibacterium denitrificans* Chol-1S, and is more preferably selected from *S*. *denitrificans* S25DH1, *S*. *denitrificans* S25DH2, *S*. *denitrificans* S25DH4, and *S*. *denitrificans* S25DH2-4, and other water-dependent alkyl hydroxylases of the same family of molybdenum-dependent dimethylsulfoxid reductase enzymes, and further optionally further comprises the use of at least one chaperone cofactor selected from the respective D-subunits thereof and related cofactors thereof, such as *S*. *denitrificans* S25-DH1 D-subunit.

Preferred is the method according to the present invention, wherein the substrate is selected from hydrocarbons, vitamin D₃, 7-dehydrocholesterol, cholest-4-en-3-one, ethylbenzene, paracymene and/or alkanes, and the substrate may be hydroxylated at primary allylic/benzylic, secondary, secondary benzylic or tertiary position.

Preferably, the beta-proteobacterium as used is *Thauera aromatica,* more preferably *Thauera aromatica* K172, heterologously overexpressing the genes encoding *S*. *denitrificans* S25DH1, *S*. *denitrificans* S25DH2, *S*. *denitrificans* S25DH4 and/or *S*. *denitrificans* S25DH2-4, together with the chaperone as required.

Preferred is the method according to the present invention, wherein said method further comprises the addition of a suitable solubilizer for the substrate, preferably a cyclodextrine, such as 2-hydroxypropyl-beta-cyclodextrine (HPCD).

Preferred is the method according to the present invention, wherein said method comprises the addition of a sub-stoichiometric amount of an electron mediator having a suitable redox-potential, such as Fe³⁺, for example K₃Fe[CN]₆, or other inorganic electron mediators that can be regenerated electrochemically. The term "suitable redox-potential" refers to a redox potential that is sufficient in order to mediate the electron flow as required for the reaction as desired, here the oxidation, and thus to produce a sufficient amount of hydroxylated substrate as described herein. In other words, it is decisive that the electrons are actually transferred between enzyme and mediator. How to determine redox-potentials is described in the literature and also known to the person of skill in the art.

Advantageously, the method according to the present invention does not produce calcitriol and/or does not produce substantial amounts of calcitriol or other undesired by-products of the hydroxylation (see also below).

The method according to the present invention provides high yields with a conversion of >75%, preferably >80%, more preferably >90%, and most preferably >95%, such as >99%.

More preferably, the method according to the present invention is performed as a continuous method, a two-phase method, and/or as a fed batch method.

In a third aspect thereof, the present invention solves the above object by providing a method for producing a biohybrid electrode for use in the bioelectrochemical hydroxylation of organic compounds, comprising the steps of a) providing a suitable hydrophilic carbon fiber electrode material, b) providing a cell culture of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase, located in the periplasm of the cell, wherein the cell culture was grown in a medium comprising an elevated defined amount of an inorganic chloride salt in order to improve the formation of a biofilm, c) injecting the cell culture of b) into the center of the biocompatible electrode, and subsequent culturing to obtain a biofilm formed on the biocompatible electrode material.

Preferred is the method according to the present invention, comprising growing *Thauera aromatica* K172 as a biofilm directly on the electrode and with elevated defined amounts of potassium and/or sodium chloride or other biofilm-inducing supplements.

In a fourth aspect thereof, the present invention solves the above object by providing a method for producing a biohybrid electrode for the bioelectrochemical hydroxylation of organic compounds, comprising the steps of a) providing a suitable biocompatible electrode comprising a material selected from an IR transparent conductive metal oxide (TCO), a carbon fiber electrode material, a glassy carbon material, or a composite electrode comprising an inorganic electrode material and a carbon fiber electrode material, and b) immobilizing at least one suitable water-dependent alkyl-hydroxylase on the material of the biocompatible electrode.

Both planar and porous electrodes can be made of carbon, inorganic materials such as metal oxides or metal oxide carbon composites. The electrodes can be easily optimized for interaction with the isolated enzyme or whole cells. Important factors for the interaction include amongst others the roughness, hydrophilicity, charge and functionalization of the electrode surface (see also above).

Preferred is the method according to the present invention, wherein the carbon electrode material comprises a paper, a woven or non-woven fabric electrode material, or a fleece.

Preferred is the method according to the present invention, wherein the immobilization between enzyme and biocompatible electrode material is provided through electrostatic interactions, coordinative binding of enzyme surface residues, such as, for example histidine moieties or tags, preferably hexa-histidine tags, and/or covalent binding of the enzyme, preferably through targeted functionalization.

In a fifth aspect thereof, the present invention solves the above object by providing a biohybrid electrode for the bioelectrochemical hydroxylation of organic compounds, produced according to a method according to the present invention.

In a sixth aspect thereof, the present invention solves the above object by providing the use of a biohybrid electrode according to the present invention for the bioelectrochemical hydroxylation of organic compounds, preferably according to a method according to the present invention.

The present invention further provides the use of a recombinant strain of a beta-proteobacterium, preferably *Thauera aromatica,* more preferably *Thauera aromatica* K172, that heterologously overexpresses the genes encoding the structural subunits of at least one functional water-dependent alkyl-hydroxylase together with at least one chaperone cofactor, preferably located in the periplasm of the cells, for the production of 25-hydroxy-vitamin D₃ (25-OH-VitD₃), 25-hydroxy-7-dehydrocholesterol (25-OH-7-DHC) and/or analogous alcohols thereof, preferably in a method according to the present invention.

As mentioned above, the present invention provides two different approaches for the bioelectrochemical hydroxylation of an organic compound.

In a first method for the bioelectrochemical hydroxylation of an organic compound according to the present invention, at least one biocompatible electrode is provided, comprising a material suitable for supporting the components involved in the bioelectrochemical hydroxylation of organic compounds. Then, at least one suitable water-dependent alkyl-hydroxylase is suitably immobilized on the biocompatible electrode. Subsequently, hydroxylation is performed as described below using the immobilized enzyme.

In a second method for the bioelectrochemical hydroxylation of an organic compound according to the present invention, at least one biocompatible electrode comprising a material suitable for supporting the components involved in the bioelectrochemical hydroxylation of organic compounds is provided. Then, either a suspension of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase, preferably located in the periplasm of the cells, wherein the suspension preferably comprises resting cells of a cell density (OD) of between about 10 and about 100, preferably about 50, is provided, and/or a biofilm on the at least one biocompatible electrode material is provided, preferably a biofilm grown directly on the material of the at least one biocompatible electrode, of the at least one recombinant strain of a suitable beta-proteobacterium, preferably *Thauera aromatica,* more preferably *Thauera aromatica* K172, that heterologously overexpresses the genes encoding for a water-dependent alkyl-hydroxylase, preferably located in the periplasm of the cells. Then, hydroxylation is performed as described below using the biofilm and/or the recombinant strain.

In the context of the present invention, the term "electrode" shall mean a biocompatible electrode, either fully or partially consisting of at least one biocompatible electrode material suitable for supporting the process and the components as used for the bioelectrochemical hydroxylation of organic compounds as described herein. In order to be suitable, the electrode material and thus the electrode must be able to function at least as a carrier for immobilized hydroxylating enzymes and/or a biofilm of suitable cells as described herein, and as the final electron acceptor in the reaction(s).

It was surprisingly found that resting (i.e. non-growing) cells of a cell density (OD) of between about 10 and about 100, preferably about 50 are better suitable for the production than at a higher cell density.

Preferred is the method according to the present invention, wherein said at least one water-dependent alkyl-hydroxylase and at least one optional chaperone cofactor are expressed from a recombinant plasmid, preferably further comprising a selection marker, such as gentamycin resistance.

Preferred is the method according to the present invention, wherein said culturing is performed in a medium, preferably a synthetic minimal medium, comprising benzoate, ethanol or acetate as electron donor and carbon-source and nitrate as electron acceptor under anaerobic conditions.

Preferred is the method according to the present invention, wherein said culturing is at about 30°C, or at between about 28-37°C. Preferred is the method according to the present invention, wherein said heterologous overexpression comprises a suitable induction of the recombinant enzymes, as is known to the person of skill.

In a next step, at least one suitable substrate to be hydroxylated by the water-dependent alkyl-hydroxylase is provided.

Then, the at least one substrate is suitably contacted with the immobilized water-dependent alkyl-hydroxylase, and the substrate is enzymatically hydroxylated, comprising an electron transfer between enzyme and electrode, preferably a direct electron transfer. Optionally, the hydroxylated substrate may be isolated and purified.

Alternatively, the at least one substrate is suitably contacted with the bacterium in the medium and/or on the electrode/the biofilm, and the substrate is enzymatically hydroxylated, comprising an electron transfer between enzyme and electrode, preferably a mediated electron transfer. Optionally, the hydroxylated substrate may be isolated and purified.

According to the present invention, any suitable substrate can be used that can be hydroxylated by enzymes. Preferred is the method according to the present invention, wherein the substrate is selected from hydrocarbons, vitamin D₃, 7-dehydrocholesterol, cholest-4-en-3-one, ethylbenzene, para-cymene and/or alkanes. The substrate may be hydroxylated at primary allylic/benzylic, secondary, secondary benzylic or tertiary position.

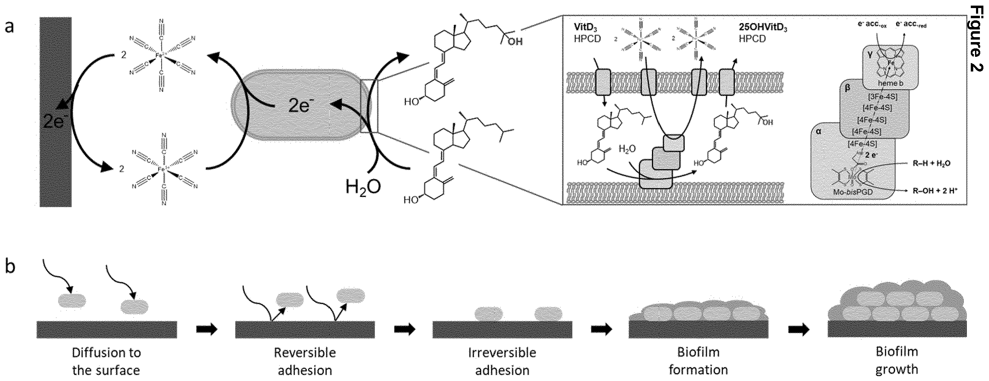

In general, any recombinant water-dependent, molybdenum-cofactor-containing alkyl-hydroxylase can be used in the methods according to the present invention, as long as it hydroxylates, and preferably selectively hydroxylates, a C-H-residue in a tertiary, secondary or primary-allylic or primary-benzylic position of a compound, such as vitamin D₃ (VitD₃) and/or 7-dehydrocholesterol (7-DHC). Preferred enzymes as used in the context of the present invention are shown in Figure 2B. The water-dependent alkyl-hydroxylase is preferably selected from an enzyme of the group steroid C25 dehydrogenase 1 (S25DH1) enzymes and related molybdoenzymes and related water-dependent alkyl-hydroxylases. All these enzymes consist of three subunits, wherein the A-subunit comprises the active site and determines both reactivity and selectivity. Thus, related enzymes in the context of the present invention are enzymes, such as bacterial enzymes, that are defined by a functional active site, molybdenum-cofactor-harboring A-subunit of the dimethyl sulfoxide reductases (DMSO) family of molybdenum-cofactor-containing hydroxylases, preferably with an amino acid sequence identity of the A-subunit of 35% or more, preferably of 50% or more, compared to the A-subunit of the enzyme S25DH1, enzymes that have the same structure (e.g. are molybdoenzymes or have the same subunit structure) and/or perform the same function, i.e. are able to hydroxylate, and preferably selectively hydroxylate, a C-H-residue in a tertiary, secondary or primary-allylic or primary-benzylic position of a compound, such as vitamin D₃ (VitD₃) and/or 7-dehydrocholesterol (7-DHC), more preferably under the conditions as described herein (see also the alkyl hydroxylases as listed in Fig. 2B). Related enzymes also include enzymes sharing an amino acid identity of more than 80%, preferably more than 90%, and even more preferred of more than 95% with the subunits of *S*. *denitrificans* S25DH 1 and/or the subunits of *S*. *denitrificans* S25DH2. Preferred are enzymes selected from *S*. *denitrificans* S25DH1, *S*. *denitrificans* S25DH2, *S*. *denitrificans* S25DH4, and *S*. *denitrificans* S25DH2-4 or related enzymes thereof as disclosed herein. Particularly preferred are enzymes selected from *S*. *denitrificans* S25DH1, *S*. *denitrificans* S25DH2, *S*. *denitrificans* S25DH4, and *S*. *denitrificans* S25DH2-4 that are mutated in order to achieve a higher hydroxylation rate of vitamin D₃ (VitD₃) and/or 7-dehydrocholesterol (7-DHC) as disclosed herein, in particular in the context of the production strain *Thauera denitrificans K172.*

In the context of the method according to the present invention, at least the D-subunit, and preferably at least one B- and/or C- subunit and at least one chaperone cofactor have to be co-expressed in the recombinant production strain, in order to provide functional and properly folded water-dependent alkyl-hydroxylases. Preferably, said at least the D-, B- and C-subunits one and at least one chaperone cofactor to be expressed is selected from the respective BCD-subunits thereof and related cofactors thereof, such as *S*. *denitrificans* S25DH1 D-subunit. Expression can be achieved using the same plasmid as for the expression of the water-dependent alkyl-hydroxylase(s), and/or a separate one. Respective plasmids are disclosed in the literature, and known by the person of skill in the art.

Preferred is the method according to the present invention, wherein said at least one water-dependent alkyl-hydroxylase subunit and at least one chaperone cofactor are expressed from a recombinant plasmid, preferably further comprising a selection marker, such as gentamycin resistance. A further preferred example is described in the example, below.

Other options for producing the enzymes and/or parts thereof as used in the context of the present invention comprise expression cassettes that are integrated into the bacterial genome. Respective elements are disclosed in the literature, and known by the person of skill in the art.

The heterologous expression or overexpression can be controlled, preferably using a suitable induction (chemically (e.g. IPTG), temperature, co-factors, genetic elements for controlling expression) of the recombinant enzymes (see above).

Particularly preferred is the method according to the present invention, wherein the hydroxylase is a steroid C25 dehydrogenase, and the hydroxylation only takes place at the C25 of the alkyl side chain of the substrate, preferably of vitamin D₃, 7-dehydrocholesterol, and/or cholest-4-en-3-one.

Preferred example for the systems used in the present invention are as follows:
Enzyme-related method:
   Preferred system as used: 2.0 mM VitD₃, 10 µM [Fe(CN)₆] as mediator, 5% HPCD, 100 µL 8 µM S25DH1 immobilized for 30 minutes. No conversion rates could be specified, but a proof of a direct electron transfer by comparing the electrochemistry before and after mediator addition was found.
Whole cell-related methods:
   Compared to the use of over-stoichiometric amounts of electron mediator (e.g. 10 mM NaNOs or [Fe(CN)₆]), a very small amount of electron mediator is sufficient to perform bioelectrochemical reactions with cells in suspension.

For the first time, in the context of the present invention it is now possible to grow biofilms on electrode materials, namely on carbon fleece electrodes, the surface of which must be oxidized beforehand in order for the interaction with the cells to take place. The addition of defined amounts of NaCl to the cell culture solution was required, and seems to be the decisive feature, in order to accelerate a biofilm formation, followed by an injection of the cell culture into the center of the fleece electrode and subsequent further cultivation.

Biofilm biohybrid electrodes require significantly fewer cells and achieve a higher turnover rate compared to cells in suspension. Biofilm: approx. 4.25 to 8.5 mg absolute protein mass (depending on electrode area) vs Suspension: approx. 123 mg absolute protein mass in suspension.

Preferred systems as used in the invention:
0.5 mM VitD₃, 4 mM [Fe(CN)₆] as electron mediator, 5% HPCD, 1 cm² planar glassy carbon electrode, potential of 600 mV vs standard hydrogen electrode (SHE) S25DH1 producing *T. aromatica* cell in suspension (123 mg protein mass in 20 ml overall volume). Turnover of approx. 83% within 20 hours.

0.5 mM VitD₃, 3 mM [Fe(CN)₆] as electron mediator, 5% HPCD, 2 cm² carbon fiber fleece electrode, potential of 600 mV vs SHE S25DH1 producing *T. aromatica* cells/biofilm on electrode in suspension (4.25 mg protein mass in 20 ml overall volume).

Turnover of approx. 80% within 20 hours

2.5 mM VitD₃, 3mM [Fe(CN)₆] as electron mediator, 25% HPCD, 4 cm² carbon fiber fleece electrode, potential of 600 mV vs SHE S25DH1 producing *T. aromatica* cell as biofilm (8.5 mg protein mass in 20 ml overall volume).

Turnover of 96% within 48 hours, whereby the amount of protein is reduced by a factor of 15 compared to WO 2022/101282A1.

As mentioned above, in case of the cells, the enzyme(s) is/are preferably located in the periplasm of the cell, which does not require an entry of the substrate into the cellular cytoplasm.

By coupling either the isolated enzyme or the S25DH-producing *Thauera aromatica* K172 whole-cell biocatalyst, either in suspension or as a biofilm, with biocompatible inorganic electrodes, unique biohybrid electrodes that can continuously synthesize 250HVitD₃ are accessible. As a result, turnover limitation due to electron acceptor consumption can be overcome by a direct or mediated electron transfer towards the electrode as the final electron acceptor.

Growing S25DH-producing *Thauera aromatica* K172 as biofilms on the electrode surface reduces the distance to be covered by the mediator between the whole cell and the electrode. A successful biofilm formation of *Thauera aromatica* K172 cells on electrodes or other surfaces has not been described in the literature. In another aspect of the method according to the present invention, the method is performed with the recombinant strain of a suitable beta-proteobacterium, preferably *Thauera aromatica,* more preferably *Thauera aromatica* K172, as a biofilm, preferably a biofilm on an electrode material.

The method according to the present invention is preferably performed with a suitable beta-proteobacterium, preferably the species *Thauera aromatica,* more preferably *Thauera aromatica* K172. Further preferred are bacteria selected from the genus *Rhodocyclales,* for example *Sterolibacterium denitrificans.* These bacteria are selected based on their suitability to be grown in culture, in a biofilm and their safety levels, e.g. level S1.

Conveniently, the method according to the present invention uses water as hydroxylating agent.

The present invention provides biohybrid-electrodes that allow for a bioelectrochemical hydroxylation without the requirement of an addition of more than stoichiometric amounts of electron acceptor, since the electrode functions as the electron acceptor. Also, limitations of the turnover due to the stoichiometric consumption of electron acceptor (e.g. 10 mM NaNO₃ or [Fe(CN)₆]) are avoided. Nevertheless, if desired, the method may comprise the addition of a sub-stoichiometric amount of an electron mediator, such as Fe³⁺, for example K₃Fe[CN]₆, or other inorganic electron mediators that can be regenerated electrochemically (see, for example, Chen H, et al. Fundamentals, Applications, and Future Directions of Bioelectrocatalysis. Chem Rev. 2020 Dec 9;120(23):12903-12993. doi: 10.1021/acs.chemrev.0c00472. Epub 2020 Oct 14. PMID: 33050699). Suitable inorganic electron mediators are known to the person of skill, and can be taken from the literature (see, for example, Martinez CM, Alvarez LH. Application of redox mediators in bioelectrochemical systems. Biotechnol Adv. 2018 Sep-Oct;36(5):1412-1423. doi: 10.1016/j.biotechadv.2018.05.005. Epub 2018 May 29. PMID: 29857046., or A. Gemünde, B. Lai, L. Pause, J. Krömer, D. Holtmann, Redox Mediators in Microbial Electrochemical Systems ChemElectroChem 2022, 9, e202200216). These mediators usually also have a suitable redox-potential (see above).

In the inventive methods using the isolated enzyme(s), the biohybrid-electrode for the first time allows for a mainly direct electron transfer between enzyme and electrode. This was not possible so far. The addition of a sub-stochiometric small amount of an electron-mediator (e.g. [Fe(CN)₆]) is possible in order to even increase the turnover rate.

Preferred is the method according to the present invention, wherein the hydroxylation produces 25-hydroxy-vitamin D₃ (25-OH-VitD₃), 25-hydroxy-7-dehydrocholesterol (25-OH-7-DHC), and/or analogous alcohols thereof.

It is a particularly advantageous feature of the method according to the present invention that it does not produce calcitriol and/or does not produce substantial amounts of calcitriol or other undesired by-products of the hydroxylation. In particular in view of the biological effects of this highly effective hormone, calcitriol is undesired in the method, and needs to be removed, which is difficult and cost intensive.

Preferred is a method according to the present invention, wherein said method further comprises the addition of a suitable solubilizer for the substrate, preferably a cyclodextrin, such as 2-hydroxypropyl-beta-cyclodextrine (HPCD), for example as disclosed in the examples, below.

It is a particularly advantageous feature of the method according to the present invention that said method provides a yield of the conversion of >75%, preferably >80%, more preferably >90%, and most preferably >95%, such as >99% (see also table 2, below). These yields are not reached by other whole cell systems as known, without that an electron acceptor was continuously added. See, for example, the recent publication of Kosian et al. (in: Highly selective whole-cell 25-hydroxyvitamin D3 synthesis using molybdenum-dependent C25-steroid dehydrogenase and cyclodextrin recycling. Microb Cell Fact 23, 30 (2024). https://doi.org/10.1186/s12934-024-02303-6), including an electron acceptor.

Preferred is a method according to the present invention, wherein said method is performed as a continuous method or a two-phase method, such as liquid-liquid or liquid-solid. Alternatively preferred is a method according to the present invention, wherein said method is performed as a (static) "fed-batch" method. This is advantageous for industrial methods for the conversion as disclosed herein. The converted 25-OH-VitD₃, 25-OH-7-DHC and/or the analogous alcohols thereof can be conveniently isolated from the medium, and consequently the whole cells can be used for an extended period of time.

Another important aspect of the present invention then relates to a method for producing a biocompatible electrode used in a method for the bioelectrochemical hydroxylation of organic compounds. The method first comprises the step of providing a suitable hydrophilic carbon fiber electrode material. This may comprise suitably oxidizing the surface of the electrodes in order to achieve an improved cell/electrode material interaction, and/or the production of already functionalized carbon fibers having sufficient hydrophilic properties, for example using polyaniline and sufficiently low temperatures in order to maintain the hydrophilic temperatures.

Thus, preferred is the method according to the present invention, further comprising the step of functionalizing the electrode material for better cell adhesion, comprising providing a predetermined surface roughness, strong acid treatments, preferably using concentrated nitric acid (HNO₃) to increase the hydrophilicity of the electrode, and/or to alter the surface charge of the electrode, preferably by doping or by functionalization of the electrode surface to improve biofilm-formation. The carbon fiber electrode material may also be functionalized with preselected and/or predetermined carbon nanoparticles in order to control/provide the hydrophilic properties and/or to create a predetermined surface roughness of the electrode material.

In the next step, a cell culture of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase is provided. In general, the culture is grown as described above, but the cell culture medium comprises an elevated defined amount of an inorganic chloride salt, such as potassium and/or sodium chloride, in order to allow/improve the formation of a biofilm on the electrode material.

Finally, the cell culture is introduced, e.g. injected into the biocompatible electrode material, for example into the center of the electrode, followed by a subsequent further culturing in order to obtain a biofilm formed on the biocompatible electrode material.

Alternatively, or cumulative, preferred is a method according to the present invention, comprising growing *Thauera aromatica* K172 as a biofilm directly on the electrode and with the same elevated defined amounts of inorganic chloride salt(s). Preferred is a method, wherein the elevated defined amount of the inorganic chloride salt is between about 0.5% wt to about 3% wt, preferably between about 0.75% wt to about 2.0% wt, more preferably at about 1.0% wt to about 1.5% wt, and most preferably at about 1 % wt. Preferred salts are potassium and/or sodium chloride. Other possible salts, amongst others, are potassium chloride, sodium chloride, calcium chloride, magnesium chloride, or zinc chloride.

In a preferred embodiment of the method according to the present invention, additionally or alternatively to the inorganic chloride salt other biofilm-inducing supplements may be used, such as extracellular polymeric substances (EPS), i.e. extracellular polysaccharides, proteins and DNA that are the major components thereof (see, for example, Flemming, HC., van Hullebusch, E.D., Neu, T.R. et al. The biofilm matrix: multitasking in a shared space. Nat Rev Microbiol 21, 70-86 (2023). https://doi.org/10.1038/s41579-022-00791-0).

In the context of the present invention, a "biofilm" shall be defined as a microbially derived sessile community characterized by cells that are irreversibly attached to a substratum or interface or each other, are embedded in a matrix of extracellular polymeric substances that they have produced, and exhibit an altered phenotype concerning growth rate and gene transcription.

Preferably, the electrode material is applied as three-dimensional mesoporous or macroporous electrode material, preferably as a macroporous electrode material, and wherein preferably the size of the pores is predetermined with respect to the size of the cells and is at between about 5 µm and about 1000 µm, preferably between about 10 µm and about 500 µm, and more preferably between about 20 µm and about 250 µm.

Another important aspect of the present invention then relates to a method for producing a biocompatible electrode material for the bioelectrochemical hydroxylation of organic compounds. Similarly to the above, the method first comprises the step of providing a suitable biocompatible electrode material comprising a material selected from an IR transparent conductive metal oxide (TCO), a carbon fiber electrode material, a glassy carbon material, or a composite electrode comprising an inorganic electrode material and a carbon fiber electrode material. Preferred is the method according to the present invention, further comprising the step of functionalizing the electrode material for better enzyme adhesion, comprising providing a predetermined surface roughness, strong acid treatments, preferably using concentrated nitric acid (HNO₃) to increase the hydrophilicity of the electrode, and/or to alter the surface charge of the electrode, preferably by doping or by functionalization of the electrode surface to improve biofilm-formation. The carbon fiber electrode material may also be functionalized with preselected and/or predetermined carbon nanoparticles in order to control/provide the hydrophilic properties and/or to create a predetermined surface roughness of the electrode material.

In the next step, the at least one suitable water-dependent alkyl-hydroxylase is immobilized on the material of the biocompatible electrode. Preferred is the method according to the present invention, wherein the material of the carbon electrode comprises a paper, a woven or non-woven fabric electrode material, or a fleece.

Preferred is the method according to the present invention, wherein the TCO is selected from a metal oxide, such as, for example, fluorine-doped tin oxide (FTO), antimony-doped tin oxide (ATO), tin-doped indium oxide (ITO), tin rich indium oxide (ITBO) or titanium dioxide (TiO₂), preferably antimony-doped tin oxide.

Further preferred is the above method according to the present invention, wherein the metal oxide is applied as part of a planar electrode, is deposited onto a carbon electrode material, and/or is applied as three-dimensional mesoporous or macroporous electrode material, preferably as a macroporous electrode material, and wherein preferably the size of the pores is predetermined with respect to the size of the enzyme, and is at between about 10 and about 1000 nm, preferably between about 40 and about 300 nm, more preferably between about 60 and about 100 nm.

Mesoporous or microporous materials are described, for example, in Li, W. et al (Mesoporous materials for energy conversion and storage devices. Nat Rev Mater 1, 16023 (2016). https://doi.org/10.1038/natrevmats.2016.23), Sharma et al. (Electrode material properties for designing effective microbial electrosynthesis systems J. Mater. Chem. A, 2019,7, 24420-24436), or Liu, Z., et al. (Three-dimensional ordered porous electrode materials for electrochemical energy storage. NPG Asia Mater 11, 12 (2019). https://doi.org/10.1038/s41427-019-0112-3).

Further preferred is the above method according to the present invention, wherein the immobilization between the enzyme and biocompatible electrode is provided through electrostatic interactions, coordinative binding of enzyme surface residues, such as, for example histidine moieties or tags, preferably hexa-histidine tags, and/or covalent binding of the enzyme, preferably through targeted functionalization.

Yet another aspect of the present invention then relates to a biocompatible electrode suitable for supporting the bioelectrochemical hydroxylation of organic compounds, produced according to the methods according to the present invention as herein.

Yet another aspect of the present invention then relates to an integration of the production methods according to the present invention for the biocompatible electrode into the bioelectrochemical hydroxylation method according to the present invention.

Yet another aspect of the present invention then relates to the use of a biocompatible electrode according to the present invention for the bioelectrochemical hydroxylation of organic compounds, preferably according to a method according to the present invention.

Yet another aspect of the present invention then relates to the use according to the present invention, wherein the hydroxylation produces 25-hydroxy-vitamin D₃ (25-OH-VitD₃), 25-hydroxy-7-dehydrocholesterol (25-OH-7-DHC) and/or analogous alcohols thereof.

Yet another aspect of the present invention then relates to the use of a recombinant strain of a beta-proteobacterium, preferably *Thauera aromatica,* more preferably *Thauera aromatica* K172, that heterologously produces at least one functional water-dependent alkyl-hydroxylase composed of the ABC-subunits and at least one chaperone cofactor, preferably located in the periplasm of the cell, for the production of 25-hydroxy-vitamin D₃ (25-OH-VitD₃), 25-hydroxy-7-dehydrocholesterol (25-OH-7-DHC) and/or analogous alcohols thereof as disclosed herein. Preferred is the use in a method according to the present invention.

The invention in a preferred embodiment relates to a synthesis of 25-hydroxy-vitamin D₃ (25-OH-VitD₃), 25-hydroxy-7-dehydrocholesterol (25-OH-7-DHC) and/or analogous alcohols from alkyl-precursors thereof as disclosed herein using whole cells, such as the preferred recombinant *Thauera aromatica* K172, heterologously expressing plasmid-encoded genes, or genes that were artificially introduced into the genome of S25DH-variants, and related water-dependent alkyl hydroxylases that serve for the selective hydroxylation of steroid-side chains as well as of other alkyl groups in tertiary, secondary, primary-benzylic or primary-allylic positions. In order to achieve an effective production, the present invention provides novel biohybrid electrode platforms for the production of 25OHVitD₃ without the need of the addition of over- or super-stoichiometric amounts of electron acceptor molecules, since the electrode itself functions as the final electron acceptor. By using S25DH from *Sterolibacterium denitrificans* Chol-1S under oxygen-excluding atmospheres instead of oxygen-dependent and NAD(P)H-consuming CYP enzymes, VitD₃ is hydroxylized specifically at the C25 position without the formation of undesired mono- or dihydroxyiated by-products such as the active hormone 1o',25(OH)₂VitD₃ (calcitriol).

Therefore, small amounts of an electron mediator are added to increase the conversion rates of VitD₃ to 25OHVitD₃, with the mediator being continuously regenerated at the electrode.

The particular use of biohybrid electrodes with whole S25DH-producing *Thauera aromatica* K172 cells avoids the expensive and time-consuming enrichment of the isolated enzyme, making the systems much more economically viable. At the same time, whole cell concepts are even more robust compared to isolated enzyme systems.

The cultivation of cells described previously and the concentration to suitable optical densities for use in whole cell biohybrid electrodes overcomes the previous limitations of stoichiometric consumption of electron acceptor molecules by recycling the electron mediator at the electrode. This also drastically reduces the required mediator concentration and enables continuous and linear formation of 250HVitD₃ over time with high productivities.

By using biohybrid electrodes consisting of biofilms of S25DH-producing *Thauera aromatica* K172 cells grown directly on the electrode surface, the number of cells required for bioelectrocatalysis is considerably reduced and the distance between the cells and the electrode surface is significantly reduced. This substantially accelerates the electron transfer between the cell and the electrode surface due to reduced diffusion paths and thus increases the efficiency of the whole cell biohybrid electrodes. Due to the continuous regeneration of the electron mediator at the electrode, only a single addition of a small amount of the mediator is required. This means that for the continuous hydroxylation of VitD₃ to 25OHVitD₃, only the substrate needs to be re-supplemented upon consumption, which further reduces the synthesis costs.

Thus, the product yields and current densities achieved on a small scale demonstrate the high efficiency of the biohybrid systems for the continuous synthesis of 250HVitD₃ and indicate potential applications of this platform in the course of up-scaling processes for even higher productivities and conversion rates.

The present biohybrid solutions are novel in terms of coupling the highly specific VitD₃ hydroxylation reaction at the C25 position by heterologous and recombinantly produced S25DH, either in form of an enriched enzyme preparation or within whole cells, with biocompatible electrode materials. This overcomes the limitations of the stoichiometric use of electron acceptor which is usually required for 250HVitD₃ synthesis.

Regarding the isolated enzyme, this is the first time that S25DH has been directly immobilized on functionalized electrodes resulting in a direct electron transfer between enzyme and electrode and thus the direct bioelectrochemical hydroxylation of VitD₃. This eliminates the need for an additional electron acceptor molecule for the established biohybrid electrode, enabling continuous and complete VitD3 hydroxylation. An electron mediator can be added to the electrochemical system to increase the conversion rate. As the mediator is continuously regenerated at the electrode, only small amounts of the mediator are needed. The approach with S25DH-producing *Thauera aromatica* K172 cells, either in suspension or in the form of a biofilm grown on an electrode, represents the first use of the whole cell catalyst in combination with an electrode as the final electron acceptor. Since the whole cells and biofilms are electrically non-conductive, the addition of an electron mediator is required to transfer electrons between the S25DH in the periplasm of the cells and the electrode.

Mediator regeneration at the electrode is possible, resulting in complete conversion of VitD₃ even in the presence of low mediator concentrations. Increasing mediator concentrations result in faster conversion rates of 250HVitD₃ formation. In contrast to whole-cell systems without electron acceptor regeneration, where the consumption of electron acceptor limits the total VitD₃ hydroxylation, product formation over time is linearly correlated with the presented biohybrid and VitD₃ can be hydroxylated continuously.

The invention relates to following items:
Item 1. A method for the bioelectrochemical hydroxylation of an organic compound, comprising a) providing at least one biocompatible electrode, b) immobilizing at least one suitable water-dependent alkyl-hydroxylase on the biocompatible electrode, c) providing at least one suitable substrate to be hydroxylated by the water-dependent alkyl-hydroxylase, d) suitably contacting the at least one substrate of c) with the immobilized water-dependent alkyl-hydroxylase of b), and enzymatically hydroxylating the substrate comprising an electron transfer between enzyme and electrode, preferably a direct electron transfer, and e) optionally, isolating said hydroxylated substrate.
Item 2. A method for the bioelectrochemical hydroxylation of an organic compound, comprising a) providing at least one biocompatible electrode, b1) providing a suspension of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell, wherein the suspension preferably comprises resting cells of a cell density (OD) of between about 10 and about 100, preferably about 50, or b2) suitably providing a biofilm, preferably a biofilm grown directly on the at least one biocompatible electrode material, of the at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell on the at least one biocompatible electrode material, c) providing at least one suitable substrate to be hydroxylated by the water-dependent alkyl-hydroxylase, d) suitably contacting the at least one substrate of c) with the bacterium of b1) and/or b2), and enzymatically hydroxylating the substrate comprising an electron transfer between enzyme and electrode, and e) optionally, isolating said hydroxylated substrate.
Item 3. The method according to Item 1 or 2, wherein water is used as a hydroxylating agent.
Item 4. The method according to any one of Items 1 to 3, wherein the substrate is selected from hydrocarbons, vitamin D₃, 7-dehydrocholesterol, cholest-4-en-3-one, ethylbenzene, para-cymene and/or alkanes.
Item 5. The method according to any one of Items 1 to 4, wherein the substrate is hydroxylated at primary allylic/benzylic, secondary, secondary benzylic or tertiary position.
Item 6. The method according to any one of Items 1 to 5, wherein the hydroxylation produces 25-hydroxy-vitamin D₃ (25-OH-VitD₃), 25-hydroxy-7-dehydrocholesterol (25-OH-7-DHC), and/or analogous alcohols thereof.
Item 7. The method according to Item 6, wherein the hydroxylase is a steroid C25 dehydrogenase and the hydroxylation only takes place at the C25 of the alkyl side chain of the substrate, preferably of vitamin D₃, 7-dehydrocholesterol, and/or cholest-4-en-3-one.
Item 8. The method according to Item 6 or 7, wherein said method does not produce calcitriol and/or does not produce substantial amounts of calcitriol or other undesired by-products of the hydroxylation.
Item 9. The method according to any one of Items 1 to 8, wherein said water-dependent alkyl-hydroxylase is obtained from *Sterolibacterium denitrificans,* preferably *Sterolibacterium* Chol-1-S steroid C25 dehydrogenase (S25DH1-4) and steroid C26 dehydrogenase (S26DH), *Aromatoleum aromaticum* preferably *Aromatoleum aromaticum* EbN1 ethylbenzene dehyrogenase (EbDH) or *Aromatoleum aromaticum* pCyN1 para-cymene dehydrogenase (CmdABC) and *Desulfococcus oleovorans* preferably *Desulfococcus oleovorans* Hxd3 alkane hydroxylase (AhyABC), preferably from *Sterolibacterium denitrificans* Chol-1S, and is more preferably selected from *S*. *denitrificans* S25DH1, *S*. *denitrificans* S25DH2, *S*. *denitrificans* S25DH4, and *S*. *denitrificans* S25DH2-4, and other water-dependent alkyl hydroxylases of the same family of molybdenum-dependent dimethylsulfoxid reductase enzymes, and further optionally further comprises the use of at least one chaperone cofactor selected from the respective D-subunits thereof and related cofactors thereof, such as *S*. *denitrificans* S25-DH1 D-subunit.
Item 10. The method according to any one of Items 1 to 9, wherein said method further comprises the addition of a suitable solubilizer for the substrate, preferably a cyclodextrine, such as 2-hydroxypropyl-beta-cyclodextrine (HPCD).
Item 11. The method according to any one of Items 1 to 10, wherein said method comprises the addition of a sub-stoichiometric amount of an electron mediator having a suitable redox-potential, such as Fe³⁺, for example K₃Fe[CN]₆, or other inorganic electron mediators that can be regenerated electrochemically.
Item 12. The method according to any one of Items 1 to 11, wherein said method provides a yield of the conversion of >75%, preferably >80%, more preferably >90%, and most preferably >95%, such as >99%.
Item 13. The method according to any one of Items 1 to 12, wherein said method is performed as a continuous method, a two-phase method, and/or as a fed batch method.
Item 14. A method for producing a biohybrid electrode, comprising the steps of a) providing a suitable hydrophilic carbon fiber electrode material, b) providing a cell culture of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell, wherein the cell culture was grown in a medium comprising an elevated defined amount of an inorganic chloride salt in order to improve the formation of a biofilm, c) injecting the cell culture of b) into the center of the biocompatible electrode, and subsequent culturing in order to obtain a biofilm formed on the biocompatible electrode material.
Item 15. The method according to Item 14, further comprising the step of functionalizing the material of the electrode for better cell adhesion, comprising providing a predetermined surface roughness, strong acid treatments, preferably using concentrated nitric acid (HNO₃) to increase the hydrophilicity of the electrode, and/or to alter the surface charge of the electrode, preferably by doping or by functionalization of the electrode surface to improve biofilm-formation.
Item 16. The method according to claim 14 or 15, comprising growing *Thauera aromatica* K172 as a biofilm directly on the electrode and with elevated defined amounts of potassium and/or sodium chloride or other biofilm-inducing supplements.
Item 17. A method for producing a biohybrid electrode, comprising the steps of a) providing a suitable biocompatible electrode comprising a material selected from an IR transparent conductive metal oxide (TCO), a carbon fiber electrode material, a glassy carbon electrode material, or a composite electrode comprising an inorganic electrode material and a carbon electrode material, and b) immobilizing at least one suitable water-dependent alkyl-hydroxylase on the biocompatible electrode.
Item 18. The method according to any one of Items 14 to 17, wherein the carbon electrode comprises a paper, a woven or non-woven fabric electrode material, or a fleece.
Item 19. The method according to any one of Items 14 to 18, wherein the carbon fiber electrode material is functionalized with predetermined carbon nanoparticles in order to predetermine the hydrophilic properties and/or to create a predetermined surface roughness of the electrode material.
Item 20. The method according to any one of Items 17 to 19, wherein the TCO is selected from a metal oxide, such as, for example, fluorine-doped tin oxide (FTO), antimony-doped tin oxide (ATO), tin-doped indium oxide (ITO), tin rich indium oxide (ITBO) or titanium dioxide (TiO₂), preferably antimony-doped tin oxide.
Item 21. The method according to Item 20, wherein the metal oxide is applied as part of a planar electrode, is deposited onto a carbon electrode, and/or is applied as three-dimensional mesoporous or macroporous electrode material, preferably as a macroporous electrode material, and wherein preferably the size of the pores is predetermined with respect to the size of the enzyme, and is at between about 10 and about 1000 nm, preferably between about 40 and about 300 nm, more preferably between about 60 and about 100 nm, or wherein preferably the size of the pores is predetermined with respect to the size of the cells and is at between about 5 µm and about 1000 µm, preferably between about 10 µm and about 500 µm, and more preferably between about 20 µm and about 250 µm.
Item 22. The method according to any one of Items 17 to 21, wherein the immobilization between enzyme and biocompatible electrode is provided through electrostatic interactions, coordinative binding of enzyme surface residues, such as, for example histidine moieties or tags, preferably hexa-histidine tags, and/or covalent binding of the enzyme, preferably through targeted functionalization.
Item 23. A biohybrid electrode for the bioelectrochemical hydroxylation of organic compounds, produced according to any one of Items 14 to 22.
Item 24. The method according to any one of Items 1 to 13, further comprising the steps of a method according to any one of Items 14 to 22 or the biohybrid electrode according to Item 23.
Item 25. Use of a biohybrid electrode according to Item 23 for the bioelectrochemical hydroxylation of organic compounds, preferably according to a method according to any one of Items 1 to 13.
Item 26. The use according to Item 25, wherein the hydroxylation produces 25-hydroxy-vitamin D₃ (25-OH-VitD₃), 25-hydroxy-7-dehydrocholesterol (25-OH-7-DHC) and/or analogous alcohols thereof.

The invention shall now be further described in the following examples with reference to the accompanying Figures, nevertheless, without being limited thereto. For the purposes of the present invention, all references as cited herein are incorporated by reference in their entireties.
Figure 1 shows a diagram of the water-dependent specific C25-hydroxylations as catalyzed by S25DH1. Hydroxylations of A, the natural substrate cholest-4-ene-3-one, B, 7-dehydrocholesterol, C, Vitamin D₃.
Figure 2 shows a schematic diagram of the enzyme/electrode or cell/electrode biohybrids according to the invention.

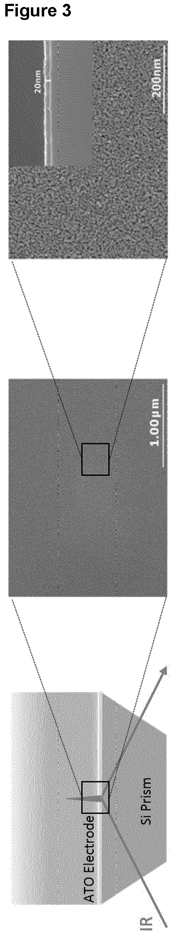

Figure 3 shows the schematic depiction of the ATO coated Si Prism for ATR-IR spectro-electrochemical experiments and SEM micrographs of the surface of the ATO layer. The inset shows a cross section of a 20 nm ATO layer on a Si waver that was prepared with the same method.

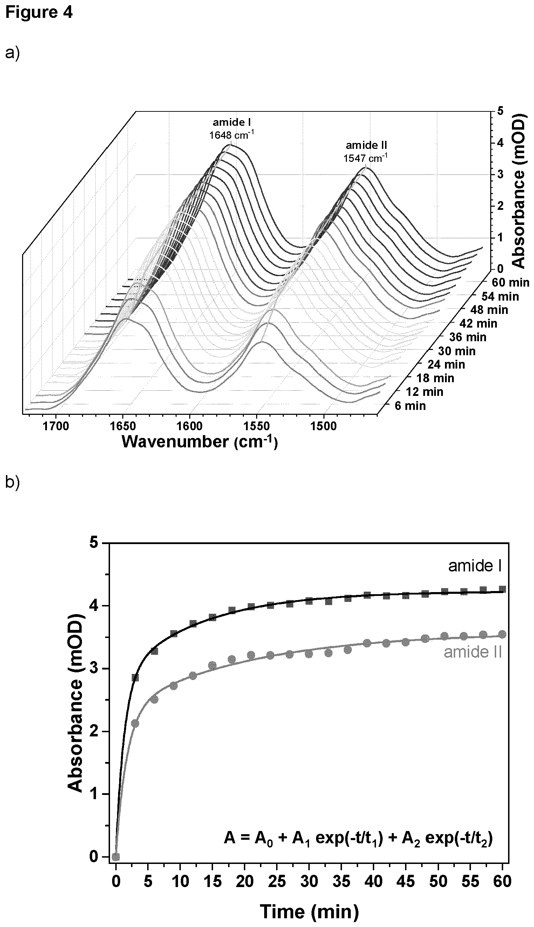

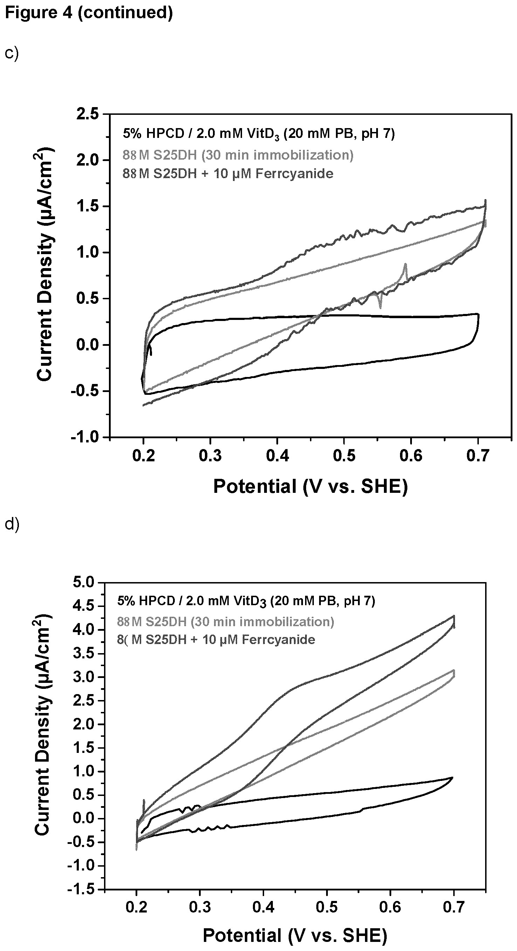

Figure 4 shows the spectro-electrochemical characterization of S25DH1 on an ATO. a) IR spectra of the amide I and amide II region dependent on the immobilization time, b) maximal peak absorbance of the amide I and amide II band dependent on the immobilization time, c) cyclic voltammogram of the blank electrode, electrode with immobilized enzyme and VitD₃ as well as electrode with immobilized enzyme, VitD₃ and ferricyanide as electron mediator for an ATO@Si electrode and d) cyclic voltammogram of the blank electrode, electrode with immobilized enzyme and VitD₃ as well as electrode with immobilized enzyme, VitD₃ and ferricyanide as electron mediator for an ATO@FTO electrode.

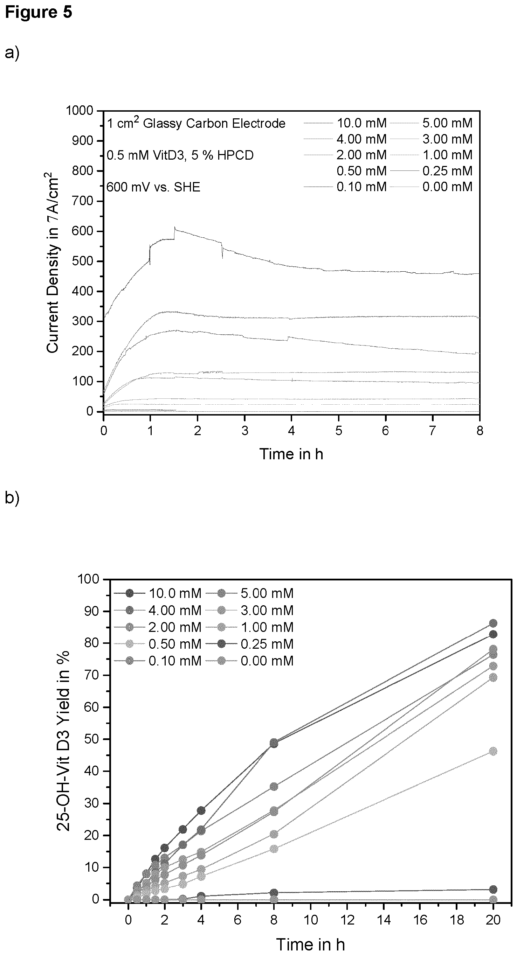

Figure 5 shows the bioelectrochemical hydroxylation of VitD₃ by S25DH-producing *Thauera aromatica* K172 cells in suspension and a glassy carbon working electrode, a) Chronoamperometry measurements for different concentrations of ferricyanide as electron mediator and b) product quantification of 25OHVitD₃.

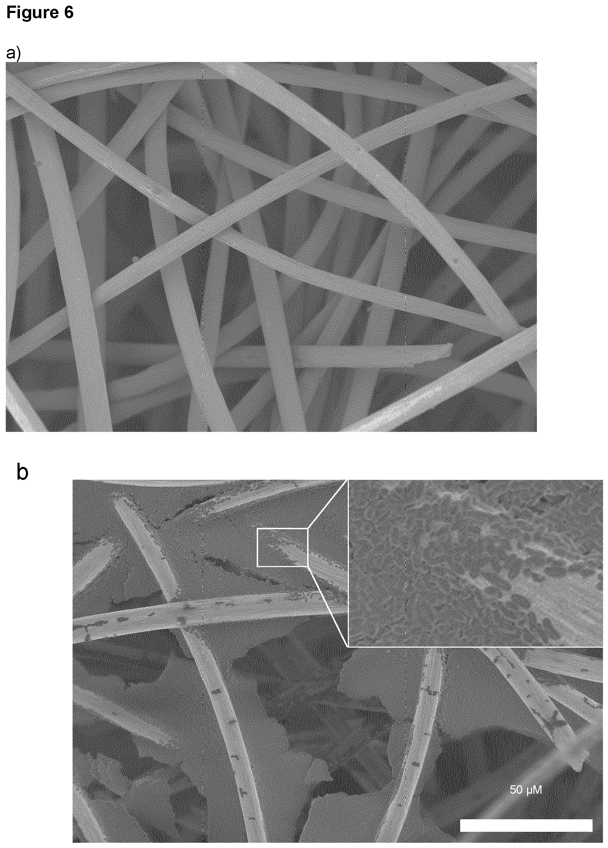

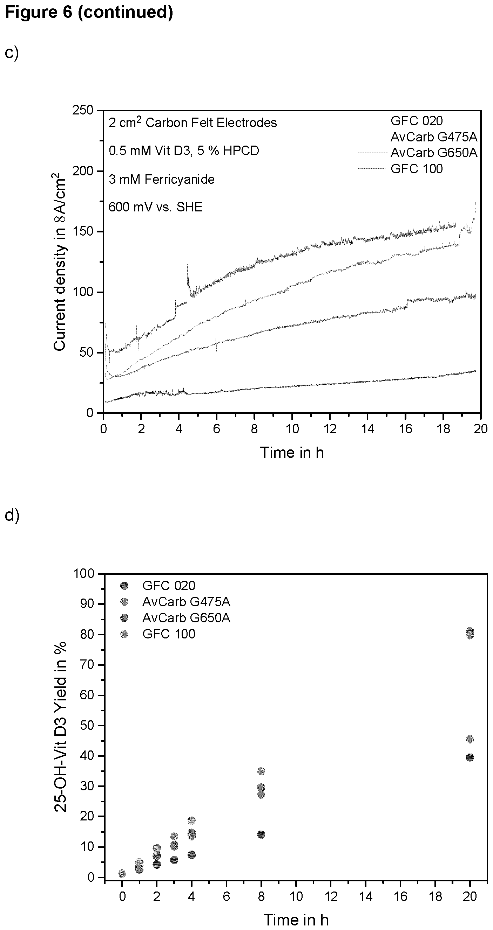

Figure 6 shows a) an SEM micrograph of a pristine carbon fiber felt and b) of a grown *Thauera aromatica* K172 biofilm on carbon fiber felt, c) Chronoamperometric measurement for four different thicknesses of carbon fiber felts (GFC020 corresponding to a felt thickness of 2.0 mm, AvCarb G475A with 4.75 mm, AvCarb G650A with 6.5 mm and GFC100 with 10 mm) and d) product quantification of 25OHVitD₃.

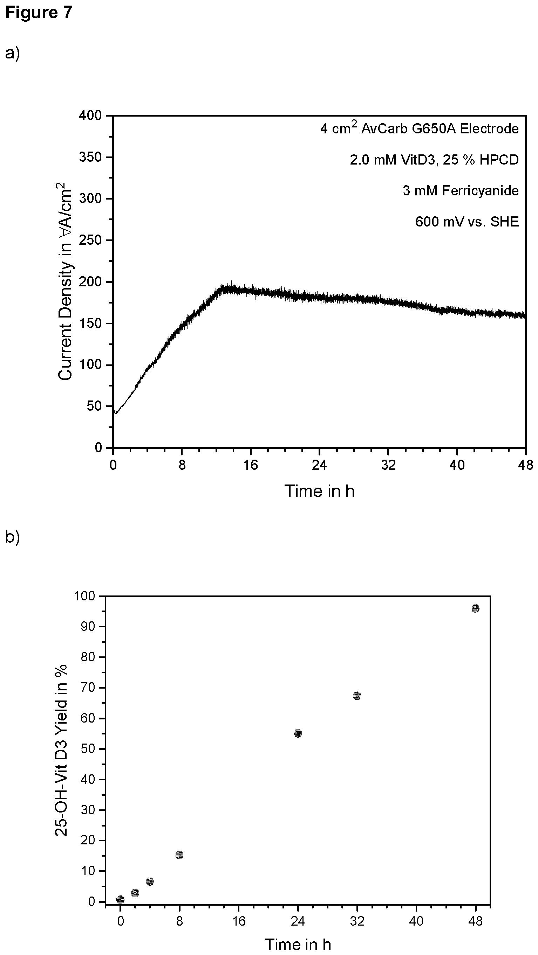

Figure 7 shows a) a chronoamperometry measurement for an optimized biofilm biohybrid electrode with high VitD₃ substrate concentration and b) product quantification of 25OHVitD₃.

### Examples

### Methods

The three structural genes of S25DH1 (Genbank Accession-No. SDENCHOL_20804, SDENCHOL_20461, and SDENCHOL_20462) as well as the gene of a chaperon (SDENCHOL_20207) were cloned into a plasmid comprising a gentamycin-resistance-cassette and transformed into the recombinant host strain *Thauera aromatica* K172 (beta-proteobacterium, safety category S1). The resulting strain was grown with benzoate as electron donor and carbon-source and nitrate as electron acceptor under anaerobic conditions.

*T. aromatica* was grown in a ,fed-batch' method with benzoate and nitrate under anaerobic conditions to an optical density of 5-6, wherein a nitrate/benzoate-mixture in a ratio of 3:1 was continuously added to the culture. Consequently, the cells grow under nitrate-limitation, hence avoiding an undesired nitrite formation. Subsequently using centrifugation and resuspension in a medium without nitrate and benzoate the cells were adjusted to an optical density of between 10-100 (Jacoby et al. 2018).

It was surprisingly found that in *Thauera aromatica* K172 ,resting-cells' that were generated as described above and heterologously produced the three genes of S25DH-variants and the chaperone as required for proper folding the following processes took place that are essential for the whole cell catalysis: (i) the transport of the substrates to be hydroxylated, such as Vitamin D₃ in the presence of 7.5% HPCD through the intact outer membrane to the heterologously produced enzyme into the periplasm (=space between outer and inner membrane), (ii) the enzymatic hydroxylation using heterologously produced S25DH with water through the transfer of electrons to acceptors in the periplasm, and (iii) the release (transport back) of the product into the culture medium. It was found in the context of this invention that hydroxylases, such as, for example S25DH1, that are heterologously produced in *T. aromatica* K172 will transfer electrons onto the cytochrome c as produced by the production strain.

### Enzyme production and enrichment for enzyme biohybrid-electrodes

S25DH1 from *Sterolibacterium denitrificans* Chol-1S was heterologously produced in the beta-proteobacterium *Thauera aromatica* K172 using an expression system under control of an IPTG-inducible promotor encoding the three subunits of the enzyme (gene identifiers: SDENCHOL_20804, SDENCHOL_20461, SDENCHOL_20462) and an essential chaperone (SDENCHOL_20207) for insertion of the molybdenum cofactor.

Enrichment was achieved via two chromatographic steps as described (Jacoby et al. 2018). In brief, cell extracts containing only soluble proteins were loaded onto a DEAE-Sepharose anion exchange column equilibrated with 20 mM Tris/H₃PO₄ (pH 7) and eluted at 150 mM KCI. The enriched S25DH1 containing protein fraction was diluted (1:10) in 20 mM MES/NaOH (pH 6) and loaded onto a Reactive Red 120-Agarose affinity column. S25DH1 was eluted by pH shift at 40% high-pH buffer (20 mM Tris/H 3 PO4, pH 8) and concentrated using centrifugal concentrators with a 30 kDa cutoff membrane. Until further use, protein samples were stored at -80 °C or in liquid nitrogen.

### Electrode preparation for enzyme immobilization

8 mol-% antimony-doped tin oxide (ATO) was synthesized as previously reported (Frasca et al. 2013). The obtained ATO nanoparticles (10 mg) and Pluronic F127 (1 mg) were dispersed in THF (1 mL) and small volumes of cone. HCl were added (~60 µL) until a colloidal solution was obtained. 30 µL of this solution was either coated onto fluorine-doped tin oxide (FTO) glass (1x2 cm, Sigma Aldrich) or undoped silicon (Si) ATR-IR prisms by spin-coating (80 rps, 10 sec). The as prepared electrodes were subsequently aged at 100 °C for 6 h and then calcined at 450 °C for 30 min (heating ramp 0.6 K min⁻¹) under air.

3D porous ATO electrodes were prepared by mixing the ATO solution with monodisperse polystyrene beads (400 nm) before spin-coating. The as prepared electrodes were aged at 100 °C for 6 h and then calcined at 450 °C for 30 min (heating ramp 0.6 K min⁻¹) under air.

Carbon paper (1x2 cm, Sigma Aldrich) or carbon felt (1x2 cm, Fuel Cell Store) electrodes were sonicated in water, ethanol and acetone for 15 minutes each. Subsequently, the electrodes were tried in argon flow. The electrodes were then used for enzyme immobilization without modification or dip coated in the previously prepared ATO solution. The ATO coated electrodes were aged at 100 °C for 6 h and then calcined at 450 °C for 30 min (heating ramp 0.6 K min⁻¹) under air.

### Enzyme immobilization and (spectro)electrochemistry

ATR-IR measurements were performed in a Kretschmann configuration using a TENSOR II IR spectrometer from Brukerwfth the Si prisms as prepared herein. Spectra were recorded between 1000 and 3500 cm⁻¹ with a resolution of 4 cm⁻¹ and an acquisition time of 3 min. Spectroelectrochemical experiments were carried out in a temperature-controlled custom-built glass cell under argon atmosphere. Enzyme immobilization was performed at 4 °C while electrochemical experiments were carried out at 25 °C.

To monitor the immobilization of S25DH1, IR-spectra of the enzyme storage buffer (20 mM Tris/H₃ PO₄, pH 7.4) were measured, averaged, and subsequently used as the background spectrum. The storage buffer was then removed, and an enzyme aliquot (200 µL, 8 µM) was injected into the cell. Spectra were recorded over a duration of 15-60 min. After immobilization, the electrode was washed three times with fresh storage buffer to remove unbound enzyme. Electrochemical investigations of the immobilized enzyme were carried out where the ATO-coated Si prism was designated as the working electrode, a platinum wire as the counter electrode and a 3.0 M KCI Ag/AgCI (0.211 V vs. SHE) electrode as the reference. Herein, 2.67 mL of fresh phosphate buffer (20 mM, pH 7) was used as the electrolyte and a suspension of VitD₃ (30 µL, 50 mM in isopropanol) in HPCD (300 µL, 50%, w/v in enzyme storage buffer) as the substrate. Cyclic voltammograms were taken between 0.2 and 0.7 V vs. SHE and at a scan rate of 2 mV s⁻¹ of the bare electrode/buffer, with immobilized enzyme and VitD₃ and then finally with ferricyanide added sequentially for each immobilization. The addition of ferricyanide as an electron mediator was used primarily to determine the dominant electron transfer mechanism (direct vs. mediated electron transfer). Electrochemical investigations of ATO coated FTO glass electrodes were performed in a one-pot electrochemical cell under same conditions using the same procedures and measurement protocols.

### Whole cell and biofilm cultivation for cell/electrode biohybrids

*Thauera aromatica* K172 containing a plasmid encoding the alpha, beta and gamma subunits of S25DH1 as well as an essential chaperone was cultivated under anaerobic conditions (2 L bottles with sealed lids) with 5 mM sodium benzoate as carbon source and 15 mM sodium nitrate as electron acceptor in a phosphate-buffered minimal medium at 30 °C as described (Jacoby et al. 2018). The minimal medium contained 5 mM NaH₂PO₄, 32 mM K₂HPO₄, 10 mM NH₄Cl, 0.1 mM CaCl₂, 1 mM MgSO₄, trace elements (1.5 mg L⁻¹ FeCl₂ × 4 H₂O, 70 µg L⁻¹ ZnCl₂, 100 µg L⁻¹ MnCl₂ × 4 H₂O, 2 µg L⁻¹ CuCl₂ × 2 H₂O, 190 µg L⁻¹ CoCl₂ × 6 H₂O, 24 µg L⁻¹ NiCl₂ × 6 H₂O, 36 µg L⁻¹ Na₂MoO₄ × 2 H₂O, 6 µg L⁻¹ H₃BO₃) and vitamins (200 µg L⁻¹ nicotinic acid, 20 µg L⁻¹ D(+)-biotin, 20 µg L⁻¹ thiamine dichloride, 80 µg L⁻¹ 4-aminobenzoic acid, 100 µg L⁻¹ Ca-D(+)-pantothenic acid, 300 µg L⁻¹ pyridoxine-dihydrochloride, 100 µg L⁻¹ cyanocobalamine) at pH 7.8 as well as 20 µg L⁻¹ gentamycin for plasmid stability and selection. At an optical density (at 578 nm, OD 578 nm) of 0.6-0.8, S25DH1 production was induced using 1 mM IPTG. Upon consumption of nitrate, the cultures were re-supplemented with sodium benzoate and sodium nitrate (5 mM and 15 mM, respectively). Cultivation was carried out until the late-exponential phase achieving OD578 nm values of 5 - 6. After reaching a resting growth state, cell suspensions were stored at 4 °C for up to four weeks, maintaining S25DH1 activity, and applied in whole cell/electrode biohybrids.

An adhesive growth as a biofilm was achieved under similar growth conditions in the presence of 1 % (w/v) NaCl. The electrodes as inorganic counterpart for the desired biofilm biohybrid electrode (3D porous carbon felts with an area of 2 or 4 cm² and varying thicknesses, Fuel Cell Store, activated by HNO₃ treatment for 4 h at 80 °C) were immersed in the growth medium and sterilized. Inoculation of the carbon felts with an exponentially growing pre-culture was achieved by direct injection into the material using a syringe with a cannula. Together with induction of S25DH1 production at OD578 nm of 0.6 - 0.8 by IPTG addition, 1 % (w/v) NaCl was added to induce adhesive growth. Growth cultures were re-supplemented with sodium benzoate (5 mM) and sodium nitrate (15 mM) daily for four subsequent days. After the final addition of growth substrates, the cells were incubated at 30 °C for a further three days to reach a resting state and then transferred to 4 °C for storage for up to four weeks. Cell adhesion was monitored using a SU8220 scanning electron microscope (SEM, Hitachi) at an accelerating voltage of 0.5 kV and a working distance of 6 mm.

### Whole cell electrochemistry

The biofilm biohybrid electrodes as prepared herein were removed from their growth medium and rinsed carefully with fresh buffer (5 mM NaH₂ PO₄, 32 mM K₂HPO₄, 10 mM NH₄Cl, pH 7.8). The electrode was then inserted in a three-electrode setup as working electrode. A platinum mesh was used as a counter electrode and an Ag/AgCI DryRef electrode was used as a reference electrode. A mixture of buffer (5 mM NaH₂PO₄, 32 mM K₂HPO₄, 10 mM NH₄Cl, pH 7.8, 8.7 mL), ferricyanide (5-500 mM, 0.2 mL) and premixed VitD₃ (50 mM, 0.1 mL) in HPCD (50 %, w:v, 1.0 mL) was added and stirred at 500 rpm. Electrochemical experiments were performed in Chronoamperometric mode by applying a potential of +600 mV vs. SHE, and measuring the resulting current.

Experiments with whole cells in suspension were performed with the same method but substituting the buffer in the electrochemical cell with a cell suspension at a given optical density and replacing the working electrode with a glassy carbon sheet (1 cm² surface area).

Samples (80 µL) for UPLC quantification of 250HVitD₃ were taken after 0, 0.5, 1.0, 1.5, 2.0, 3.0, 4.0, 8.0 and 20 hours and quenched directly in isopropyl alcohol (320 µL) after collection. After all samples for a given experiment were collected, the solid components of the samples were separated by centrifugation (14.000 rpm, 20 min) twice.

### UPLC-based quantification of 250HVitDs

Samples of electrochemical experiments for the formation of 250HVitD₃ using the presented biofilm biohybrid electrodes were analyzed by ultra-performance liquid chromatography (UPLC) on an H-Class UPLC system (Waters Corporation). Separation was achieved using an ACQUITY CSH C18 column (1.7 µm, 2.1 × 200 mm) in a gradient of acetonitrile + 0.1% (v/v) formic acid and water + 0.1% (v/v) formic acid at a flow rate of 0.4 mL min⁻¹ and column temperature of 50 °C. Samples (5 µL) were applied at 50% acetonitrile + 0.1% (v/v) formic acid and eluted in an isocratic gradient of 100% (v/v) acetonitrile + 0.1% (v/v) formic acid for 4 min. Substrate and product were detected at 260 nm via UV/VIS spectroscopy and quantified by comparison of peak areas with those of authentic standards (concentration range 10-500 µM).

### Literature as cited

Abdulmughni, Ammar, et al. 2021. "Improvement of the 25-Hydroxyvitamin D3 Production in a CYP109A2-Expressing Bacillus Megaterium System." Journal of Biotechnology 325 (May): 355-59. https://doi.Org/1 0. 1 01 6/j.jbiotec.2020.09.027.
Amrein, Karin, et al. 2020. "Vitamin D Deficiency 2.0: An Update on the Current Status Worldwide." European Journal of Clinical Nutrition 74 (1 1): 1498-1513. https://doi.Org/10. 1 038/s41430-020-0558-y.
Babot, Esteban D., et al. 2015. "Steroid Hydroxylation by Basidiomycete Peroxygenases: A Combined Experimental and Computational Study." Applied and Environmental Microbiology 81 (12): 4130 12. https://doi.org/10.1128/AEM.00660-15.
Bonrath, Werner, et al. 2021. "From Sugars to Nutritional Products -Active Ingredients." Chimia 75 (9): 757-65. https://doi.org/10.2533/chimia.2021.757.
Bouillon, Roger, et al. 2019. "Skeletal and Extraskeletal Actions of Vitamin D: Current Evidence and Outstanding Questions." Endocrine Reviews 40 (4): 1109-51. https://doi.Org/1 0. 1 21 0/ER.201 8-00126.
Bouillon, Roger, and Jose Manuel Quesada Gomez. 2023. "Comparison of Calcifediol with Vitamin D for Prevention or Cure of Vitamin D Deficiency." The Journal of Steroid Biochemistry and Molecular Biology 228 (April): 106248. https://d0i.0rg/l 0. 1 01 6/j.jsbmb.2O23. 1 06248.
Cheng, Jeffrey B., et al. 2003. "De-Orphanization of Cytochrome P450 2R1." Journal of Biological Chemistry 278 (39): 38084-93. https://doi.Org/1 0. 1 074/j be. m 307028200. Dermer, Juri, and Georg Fuchs. 2012. "Molybdoenzyme That Catalyzes the Anaerobic Hydroxylation of a Tertiary Carbon Atom in the Side Chain of Cholesterol." Journal of Biological Chemistry 287 (44): 36905-16. https://doi.org/10.1074/jbc.M112.407304.
Escrib6-Gelonch, Marc, Alexei Halpin, Timothy Noel, and Volker Hessel. 2018. "Laser-Mediated Photo-High-p,T Intensification of Vitamin D3 Synthesis in Continuous Flow." ChemPhotoChem 2 (10): 922-30. https://doi.org/10.1002/cptc.201800102.
Frasca, Stefano, et al. 2013. "Bioelectrocatalysis at Mesoporous Antimony Doped Tin Oxide Electrodes-Electrochemical Characterization and Direct Enzyme Communication." Electrochimica Acta 110 (November): 172-80. https://doi.org/10.1016/j electacta.2013.03.144.
Fu, Bing, et al. 2022. "Enhancing the Production of Physiologically Active Vitamin D3 by Engineering the Hydroxylase CYP105A1 and the Electron Transport Chain." World Journal of Microbiology and Biotechnology 38 (1): 1-10. https://doi.org/10.1007/s11274-021-03193-1.
Holick, Michael F. 2017. "The Vitamin D Deficiency Pandemic: Approaches for Diagnosis, Treatment and Prevention." Reviews in Endocrine and Metabolic Disorders 18 (2): 153-65. https://doi.Org/1 0. 1 007/sl 1 1 54-017-9424-1.
Jacoby, Christian, et al. 2018. "Four Molybdenum-Dependent Steroid C-25 Hydroxylases: Heterologous Overproduction, Role in Steroid Degradation, and
Application for 25-Hydroxyvitamin D3 Synthesis." Edited by Markus W. Ribbe. MBio 9 (3): 1-14. https://doi.org/10.1128/mBio.00694-18.
Kalimuthu, Palraj, Agnieszka M. Wojtkiewicz, Maciej Szaleniec, and Paul V. Bernhardt. 2018. "Electrocatalytic Hydroxylation of Sterols by Steroid C25 Dehydrogenase from Sterolibacterium Denitrif leans." Chemistry - A European Journal 24 (30): 7710-17. https://d0i.0rg/l 0. 1 002/chem.201 80061 6.
Kametani, Tetsuji, and Hiroko Furuyama. 1987. "Synthesis of Vitamin D3 and Related Compounds." Medicinal Research Reviews 7 (2): 147-71. https://d0i.0rg/l 0. 1 002/med. 261 0070202.
Kang, Dae Jung, et al. 2015. "Bioconversion of Vitamin D3 to Calcifediol by Using Resting Cells of Pseudonocardia Sp." Biotechnology Letters 37 (9): 1895-1904. https://doi.org/10.1007/s10529-015-1862-9.
Kosian D, Willistein M, Weßbecher R, Eggers C, May O, Boll M. Highly selective whole-cell 25-hydroxyvitamin D3 synthesis using molybdenum-dependent C25-steroid dehydrogenase and cyclodextrin recycling. Microb Cell Fact. 2024 Jan 20;23(1):30. doi: 10.1186/s12934-024-02303-6. PMID: 38245746; PMCID: PMC10799449.
Lütke-Oörhoff, Michael, et al. 2022. "Dietary Supplementation of 25-Hydroxycholecalciferol as an Alternative to Cholecalciferol in Swine Diets: A Review." Journal of Animal Physiology and Animal Nutrition 106 (6): 1288-1305. https://doi.org/10.1111/JPN. 13768.
Mie, Yasuhiro, et al. 2020. "Electrochemically Boosted Cytochrome P450 Reaction That Efficiently Produces 25-Hydroxyvitamin D3." Journal of Catalysis 384: 30-36. https://d0i.0rg/l 0. 1 01 6/j.jcat.2020.02.012.
Quesada-Gomez, J. M., and R. Bouillon. 2018. "Is Calcifediol Better than Cholecalciferol for Vitamin D Supplementation?" Osteoporosis International 29 (8): 1697-1711. https://doi.Org/1 0. 1 007/s001 98-01 8-4520-y.
Rugor, A., et al. 2017. "Regioselective Hydroxylation of Cholecalciferol, Cholesterol and Other Sterol Derivatives by Steroid C25 Dehydrogenase." Applied Microbiology and Biotechnology 101 (3): 1163-74. https://doi.org/10.1007/s00253-016-7880-2.
Schmitz, Lisa Marie, et al 2021. "Investigation of Vitamin D2 and Vitamin D3 Hydroxylation by Kutzneria Albida." ChemBioChem 22 (13): 2266-74. https://doi.org/10.1002/cbic.202100027.
Tang, Dandan, et al. 2020. "Efficient Biotransformation of Vitamin D3 to 25-Hydroxyvitamin D3 by a Newly Isolated Bacillus Cereus Strain." Applied Microbiology and Biotechnology 104 (2): 765-74. https://doi.Org/1 0. 1 007/S00253-01 9-10250-1.
Vazquez, J. R., et al. 2018. "Effects of 25-Hydroxycholecalciferol with Two D3 Vitamin Levels on Production and Immunity Parameters in Broiler Chickens." Journal of Animal Physiology and Animal Nutrition 102(1): e493-97. https://doi.org/10.1111/JPN.12715.
Warnke, Markus, et al. 2016. "25-Hydroxyvitamin D3 Synthesis by Enzymatic Steroid Side-Chain Hydroxylation with Water." Angewandte Chemie International Edition 55 (5): 1881-84. https://d0i.0rg/l 0. 1 002/anie.201 510331.
Yasutake, Yoshiaki, et al. 2013. "A Single Mutation at the Ferredoxin Binding Site of P450 Vdh Enables Efficient Biocatalytic Production of 25-Hydroxyvitamin D3." ChemBioChem 14 (17): 2284-91. https://doi.Org/1 0. 1 002/cbic.201 300386.
Zhu, GD and Okumara WH (1995) synthesis of vitamin D (calciferol), Chem. Rev. 1995, 95, 6, 1877-1952.

## Claims

1. A method for the bioelectrochemical hydroxylation of an organic compound, comprising
a) providing at least one biocompatible electrode,
b) immobilizing at least one suitable water-dependent alkyl-hydroxylase on the biocompatible electrode,
c) providing at least one suitable substrate to be hydroxylated by the water-dependent alkyl-hydroxylase,
d) suitably contacting the at least one substrate of c) with the immobilized water-dependent alkyl-hydroxylase of b), and enzymatically hydroxylating the substrate comprising an electron transfer between enzyme and electrode, preferably a direct electron transfer, and
e) optionally, isolating said hydroxylated substrate,
wherein preferably water is used as a hydroxylating agent.

2. A method for the bioelectrochemical hydroxylation of an organic compound, comprising
a) Providing at least one biocompatible electrode,
b1) providing a suspension of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell, wherein the suspension preferably comprises resting cells of a cell density (OD) of between about 10 and about 100, preferably about 50, or
b2) suitably providing a biofilm, preferably a biofilm grown directly on the at least one biocompatible electrode material, of the at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell on the at least one biocompatible electrode,
c) providing at least one suitable substrate to be hydroxylated by the water-dependent alkyl-hydroxylase,
d) suitably contacting the at least one substrate of c) with the bacterium of b1) and/or b2), and enzymatically hydroxylating the substrate comprising an electron transfer between enzyme and electrode, and
e) optionally, isolating said hydroxylated substrate,
wherein preferably water is used as a hydroxylating agent.

3. The method according to claim 1 or 2, wherein the substrate is selected from hydrocarbons, vitamin D₃, 7-dehydrocholesterol, cholest-4-en-3-one, ethylbenzene, para-cymene and/or alkanes, and preferably wherein the substrate is hydroxylated at primary allylic/benzylic, secondary, secondary benzylic or tertiary position.

4. The method according to any one of claims 1 to 3, wherein the hydroxylation produces 25-hydroxy-vitamin D₃(25-OH-VitD₃), 25-hydroxy-7-dehydrocholesterol (25-OH-7-DHC), and/or analogous alcohols thereof, and wherein preferably the hydroxylase is a steroid C25 dehydrogenase and the hydroxylation only takes place at the C25 of the alkyl side chain of the substrate, preferably of vitamin D₃, 7-dehydrocholesterol, and/or cholest-4-en-3-one.

5. The method according to claim 4, wherein said method does not produce calcitriol and/or does not produce substantial amounts of calcitriol or other undesired by-products of the hydroxylation.

6. The method according to any one of claims 1 to 5, wherein said water-dependent alkyl-hydroxylase is obtained from *Sterolibacterium denitrificans,* preferably *Sterolibacterium* Chol-1-S steroid C25 dehydrogenase (S25DH1-4) and steroid C26 dehydrogenase (S26DH), *Aromatoleum aromaticum* preferably *Aromatoleum aromaticum* EbN1 ethylbenzene dehyrogenase (EbDH) or *Aromatoleum aromaticum* pCyN1 para-cymene dehydrogenase (CmdABC) and *Desulfococcus oleovorans* preferably *Desulfococcus oleovorans* Hxd3 alkane hydroxylase (AhyABC), preferably from *Sterolibacterium denitrificans* Chol-1S, and is more preferably selected from *S*. *denitrificans* S25DH1, *S*. *denitrificans* S25DH2, *S*. *denitrificans* S25DH4, and *S*. *denitrificans* S25DH2-4, and other water-dependent alkyl hydroxylases of the same family of molybdenum-dependent dimethylsulfoxid reductase enzymes, and further optionally further comprises the use of at least one chaperone cofactor selected from the respective D-subunits thereof and related cofactors thereof, such as *S*. *denitrificans* S25-DH1 D-subunit.

7. The method according to any one of claims 1 to 6, wherein said method further comprises the addition of a suitable solubilizer for the substrate, preferably a cyclodextrine, such as 2-hydroxypropyl-beta-cyclodextrine (HPCD).

8. The method according to any one of claims 1 to 7, wherein said method comprises the addition of a sub-stoichiometric amount of an electron mediator having a suitable redox-potential, such as Fe³⁺, for example K₃Fe[CN]₆, or other inorganic electron mediators that can be regenerated electrochemically.

9. A method for producing a biohybrid electrode, comprising the steps of
a) providing a suitable hydrophilic carbon fiber electrode,
b) providing a cell culture of at least one recombinant strain of a suitable beta-proteobacterium, preferably *Thauera aromatica,* more preferably *Thauera aromatica* K172, that heterologously overexpresses the genes encoding for a water-dependent alkyl-hydroxylase located in the periplasm of the cell, wherein the cell culture was grown in a medium comprising an elevated defined amount of an inorganic chloride salt in order to improve the formation of a biofilm,
c) injecting the cell culture of b) into the center of the biocompatible electrode, and subsequent culturing in order to obtain a biofilm formed on the material of the biocompatible electrode, optionally further comprising the step of functionalizing the material of the electrode for better cell adhesion, comprising providing a predetermined surface roughness, strong acid treatments, preferably using, for example comprising boiling in, concentrated nitric acid (HNO₃) to increase the hydrophilicity of the electrode, and/or to alter the surface charge of the electrode, preferably by doping or by functionalization of the electrode surface to improve biofilm-formation.

10. The method according to claim 9, comprising growing *Thauera aromatica* K172 as a biofilm directly on the electrode and with elevated defined amounts of potassium and/or sodium chloride or other biofilm-inducing supplements.

11. A method for producing a biohybrid electrode, comprising the steps of
a) providing a suitable biocompatible electrode comprising a material selected from an IR transparent conductive metal oxide (TCO), a carbon fiber electrode material, a glassy carbon electrode material, or a composite electrode comprising an inorganic electrode material and a carbon electrode material, and
b) immobilizing at least one suitable water-dependent alkyl-hydroxylase on the biocompatible electrode.

12. The method according to any one of claims 9 to 11, wherein the carbon electrode material comprises a paper, a woven or non-woven fabric electrode material, or a fleece, and/or wherein the carbon fiber electrode material is functionalized with predetermined carbon nanoparticles in order to predetermine the hydrophilic properties and/or to create a predetermined surface roughness of the electrode material.

13. The method according to claim 11 or 12, wherein the TCO is selected from a metal oxide, such as, for example, fluorine-doped tin oxide (FTO), antimony-doped tin oxide (ATO), tin-doped indium oxide (ITO), tin rich indium oxide (ITBO) or titanium dioxide (TiO₂), preferably antimony-doped tin oxide, wherein preferably the metal oxide is applied as part of a planar electrode, is deposited onto a carbon electrode, and/or is applied as three-dimensional mesoporous or macroporous electrode material, preferably as a macroporous electrode material, and wherein preferably the size of the pores is predetermined with respect to the size of the enzyme, and is at between about 10 and about 1000 nm, preferably between about 40 and about 300 nm, more preferably between about 60 and about 100 nm, or wherein preferably the size of the pores is predetermined with respect to the size of the cells and is at between about 5 µm and about 1000 µm, preferably between about 10 µm and about 500 µm, and more preferably between about 20 µm and about 250 µm.

14. A biohybrid electrode for the bioelectrochemical hydroxylation of organic compounds, produced according to any one of claims 9 to 13.

15. Use of a biohybrid electrode according to claim 14 for the bioelectrochemical hydroxylation of organic compounds, preferably according to a method according to any one of claims 1 to 8, wherein preferably the hydroxylation produces 25-hydroxy-vitamin D₃ (25-OH-VitD₃), 25-hydroxy-7-dehydrocholesterol (25-OH-7-DHC) and/or analogous alcohols thereof.
